(19) Europäisches Patentamt

European Patent Office

Office européen des brevets

(11) **EP 4 169 397 B1**

(12) **EUROPEAN PATENT SPECIFICATION**

(45) Date of publication and mention
of the grant of the patent:
**08.04.2026 Bulletin 2026/15**

(21) Application number: **22801948.5**

(22) Date of filing: **23.06.2022**

(51) International Patent Classification (IPC):
**A24F 42/20** *(2020.01)* **A61K 9/00** *(2006.01)*
**A24F 40/10** *(2020.01)* **A24F 40/42** *(2020.01)*
**A24F 40/48** *(2020.01)*

(52) Cooperative Patent Classification (CPC):
**A24F 40/42; A24F 40/48; A24F 42/20; A61K 9/008;**
A24F 40/10

(86) International application number:
**PCT/KR2022/008917**

(87) International publication number:
**WO 2023/022352 (23.02.2023 Gazette 2023/08)**

(54) **AEROSOL INHALER COMPRISING NICOTINE TRANSITION PART**

AEROSOLINHALATOR MIT NIKOTINÜBERGANGSTEIL

INHALATEUR D'AÉROSOL COMPRENANT UNE PARTIE DE TRANSITION DE NICOTINE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.08.2021 KR 20210109832**

(43) Date of publication of application:
**26.04.2023 Bulletin 2023/17**

(73) Proprietor: **KT & G Corporation
Daejeon 34337 (KR)**

(72) Inventors:
• **Jung, Yong Mi
Yuseong-gu, Daejeon 34128 (KR)**
• **Lee, Mi Jeong
Yuseong-gu, Daejeon 34128 (KR)**
• **Jeoung, Eun Mi
Yuseong-gu, Daejeon 34128 (KR)**
• **Shin, Jun Won
Yuseong-gu, Daejeon 34128 (KR)**

(74) Representative: **Ter Meer Steinmeister & Partner
Patentanwälte mbB
Nymphenburger Straße 4
80335 München (DE)**

(56) References cited:
WO-A1-2011/147699 WO-A2-2012/045683
KR-A- 20130 111 240 KR-A- 20200 145 152
KR-A- 20210 043 301 KR-B1- 101 724 522
US-A1- 2011 250 324

## Description

## Technical Field

The present invention relates to an aerosol inhaler including a nicotine transition unit.

## Background Art

Inhaler devices including an electronic cigarette that a liquid containing a nicotine solution is heated and vaporized, and the vaporized aerosol is inhaled are being developed. Many users smoke through the developed inhaler devices and the number of users who use inhaler devices is increasing. In the relevant field, a new type of aerosol inhaler, which improves conventional smoking articles to aerosolize nicotinergic substances in a manner other than burning or heating, is being developed. For example, Korea Patent No. 10-1257597 discloses a non-heating type tobacco flavor inhaler.

A propellant is a substance that carries nicotinergic substances as a nicotine supply source or aerosols generated by reacting nicotinergic substances with organic acid substances into the user's mouth, and inhalers that utilizes a propellant can be operated in a non-heating type and a non-electronic type, so that there are several advantages such as having simple structure, not using a battery, etc. When intending to inhale a nicotine liquid medium into a human body, a technique of delivering, using a propellant, an appropriate amount of nicotine to every puff may be important.

In the case of an inhaler using a propellant, most motions thereof may be determined by the propellant, and thus it is difficult to control the speed or pressure and flow rate with a device (particularly, a valve structure). Accordingly, a study on a method for controlling the transition amount of nicotine in aerosol are continuously conducted in the relevant technical field.

WO 2012/045683 A2 discloses an aerosol generator comprising a storage chamber, a dosing chamber, an expansion chamber and an orifice separating the expansion chamber from mouthpiece.

### Prior Art Document

(Patent Document)

(Patent document 1) KR20010080091A

## Disclosure

## Technical Problem

In order to solve the above problems in an aerosol inhaler using a propellant, the present invention provides an aerosol inhaler including a nicotine transition unit capable of predicting the amount of nicotine transition and controlling the amount of actual nicotine transition so as not to significantly deviate from the corresponding predicted value.

## Technical Solution

The technical problems are solved by the subject-matter of the independent claim.

According to a first aspect of the present disclosure, the present invention provides an aerosol inhaler comprising a nicotine transition unit, wherein the nicotine transition unit includes a first container filled with a nicotine-containing solution and a propellant, and a second container connected to the first container to be filled with the liquid of the first container, and the propellant exists in a liquid state and a gas state in the first container, wherein an internal space of the second container is contracted and relaxed according to the amount of the liquid filled in the first container.

In an embodiment of the present invention, the propellant is a substance having vapor pressure of 413.7 kPa relative to ambient atmospheric pressure to 68.5 kPa relative to ambient atmospheric pressure at 21°C.

In an embodiment of the present invention, the nicotine-containing solution in the first container is filled in an amount of 1% to 10% by weight based on the total weight of the propellant.

In an embodiment of the present invention, the volume of the liquid including the nicotine-containing solution and the liquid propellant in the first container is 85% by volume or less based on an internal volume of the first container.

In an embodiment of the present invention, the gas propellant, immediately before the second container is filled with the liquid after the first container is filled with the nicotine-containing solution and the propellant, contains 5% by weight or less based on the total weight of the propellant.

In an embodiment of the present invention, the second container is filled with the liquid of the first container, and is separated from the first container after the inside completely filled.

In an embodiment of the present invention, the aerosol inhaler further includes an aerosol generator, and the aerosol generator is connected to the second container separated from the first container, is supplied with the liquid filled in the second container, and generates aerosol until the liquid is all exhausted.

In an embodiment of the present invention, the liquid filled in the second container is such an amount that the aerosol generated by the aerosol generator can be all exhausted by 5 puffs to 15 puffs.

In an embodiment of the present invention, when the liquid filled in the second container is all exhausted, the second container is separated from the aerosol generator, connected to the first container, and filled with the liquid of the first container, and the repeating process of filling and exhausting the liquid is stopped when the volume of the liquid in the first container is 15% by volume or less based on the internal volume of the first container.

In an embodiment of the present invention, the liquid in the first container is such an amount that the second container can be filled 15 times to 25 times until the filling is stopped.

## Advantageous Effects

The aerosol inhaler according to an embodiment of the present invention includes a nicotine transition unit described below, thereby solving the problem in that the amount of nicotine transition is significantly changed whenever the user uses the aerosol inhaler, and controlling the change in the amount of nicotine transition within 5% or less until the aerosol inhaler is all used up.

## Description of Drawings

FIG. 1 is a view schematically showing the structure of a nicotine transition unit according to an embodiment of the present invention.

FIG. 2 is a view schematically showing the structure of an aerosol inhaler including the nicotine transition unit according to an embodiment of the present invention.

## Best Mode

Hereinafter, embodiments are described in detail with reference to exemplary drawings. When adding reference numerals to the components of each drawing, it should be noted that the same components are given the same reference numerals as much as possible even if they are shown on different drawings. In addition, in the description of the embodiment, if it is determined that a detailed description of a related known configuration or function may interfere with the understanding of the embodiment, the detailed description thereof will be omitted.

Further, the terms such as 'first', 'second', 'A', 'B', '(a)', and '(b)' may be used for describing the components of the embodiment. These terms are only for distinguishing the components from other components, and do not limit the essence, sequence, or order of the components. When a component is described as being "connected", "combined", or "coupled" to another component, it should be understood that although the component may be directly connected or coupled to another component, other components may also be "connected", "combined", or "coupled" between each component.

Components included in any one embodiment and components having a common function will be described using the same names in other embodiments. Unless otherwise described, descriptions described in any one embodiment may be applied to other embodiments as well, and detailed descriptions thereof within the overlapping range will be omitted.

The present invention relates to an aerosol inhaler including a nicotine transition unit, and aims to provide an aerosol inhaler capable of uniformly transitioning nicotine through the nicotine transition unit in an inhaler using a propellant for effectively transitioning nicotine. The term "aerosol inhaler" as used herein refers to a device that aerosolizes a nicotine-containing solution to be described later to deliver it into the user's lung through the user's mouth.

The aerosol inhaler according to an embodiment of the present invention includes a nicotine transition unit, and the nicotine transition unit includes a first container filled with a nicotine-containing solution and a propellant, and a second container connected to the first container to be filled with the liquid of the first container.

The term 'nicotine-containing solution" as used herein refers to a liquid containing a predetermined level of nicotine, the components constituting the liquid other than nicotine are alcohol, perfumery ingredients, etc., and a substance commonly used in the relevant field may be used. The nicotine may be supplied in the form of a nicotinergic substance including nicotine, nicotine salt, nicotine alkaloid, nicotine derivative, and the like, and may be effectively dispersed in alcohol contained in a large amount in the nicotine-containing solution. The alcohol is not particularly limited as long as it is commonly used in the relevant field. However, according to an embodiment of the present invention, the alcohol is selected from a group consisting of monohydric alcohol, dihydric alcohol, trihydric alcohol, and a combination thereof, wherein the monohydric alcohol is ethanol, glycol or glycol ether that is the dihydric alcohol is selected from a group consisting of propylene glycol, polypropylene glycol, polyethylene glycol, and a combination thereof, and the trihydric alcohol is glycerol. The total weight of nicotine contained in the nicotine-containing solution may be controlled according to standards and product specifications in the relevant field, but according to an embodiment of the present invention, the concentration of nicotine in the nicotine-containing solution is 1% to 10% by weight, specifically, 1.5% to 8% by weight, and more specifically 2% to 6% by weight. Aerosol generated within the above concentration range of nicotine may increase the user's feel of satisfaction.

A propellant serves to carry aerosol generated from the nicotine-containing solution to the user's mouth, and a compound that fundamentally does not cause side effects in a human body is used as a propellant. The propellant exists in a liquid state or a gas state by pressure in the first container, and when the liquid propellant is uniformly mixed with the nicotine-containing solution, and thus the liquid in the first container is filled into the second container, the liquid having composition uniform with the remainder in the first container is filled into the second container. The propellant may be selected from substances having a predetermined level of vapor pressure to be separated into a liquid state or a gas state in the first container, and these substances should have low affinity for the nicotine-containing solution so that the intrinsic functionality of the propellant may be maintained when generating an aerosol thereafter. According to an embodiment of the present invention, the propellant is a substance having a vapor pressure of 413.7 kPa relative to ambient atmospheric pressure to 689.5 kPa relative to ambient atmospheric pressure, specifically, 448.2 kPa relative to ambient atmospheric pressure to 655.0 kPa relative to ambient atmospheric pressure, and more specifically 482.6 kPa relative to ambient atmospheric pressure to 620.5 kPa relative to ambient atmospheric pressure, at 21°C. If the propellant has a vapor pressure lower than the above range, the function to carry an aerosol may be reduced, and if the propellant has a vapor pressure higher than the above range, excessive pressure is required to liquefy above a predetermined level. According to an embodiment of the present invention, the propellant is fluorinated alkane, specifically, fluorinated ethane, fluorinated propane, or fluorinated butane, and more specifically, 1,1,1,2-tetrafluoroethane or 1, 1, 1,2,3,3,3-heptafluoropropane. Specific conditions of propellants for inhalation, etc. may follow the standards of the U.S. Pharmacopeia (USP).

The ratio of the nicotine-containing solution and the propellant filled in the first container may be adjusted so that the nicotine-containing solution and the propellant can maintain their respective functionality when the liquid is filled in the second container. According to an embodiment of the present invention, in the first container, the nicotine-containing solution is filled in an amount of 1% to 10% by weight, specifically 1% to 7% by weight, and more specifically 1% to 4% by weight, based on the total weight of the propellant. If the content of the nicotine-containing solution is lower than the above range, the content of nicotine supplied to a puff once when using the aerosol inhaler may be insufficient, and if the content of the nicotine-containing solution is higher than the above range, the nicotine transition may not be effectively proceeded due to insufficient propellant content.

To completely liquefy the propellant, which is gas at room temperature, since excessive pressure is required for the first container and the first container is more easily damaged by external shock, and thus the liquid inside may leak, it is preferable to manage the liquid in the first container so as not to exceed a predetermined level. According to an embodiment of the present invention, the volume of the liquid containing the nicotine-containing solution and the liquid-state propellant in the first container is 85% by volume or less, specifically 83% by volume or less, and more specifically 81% by volume or less, based on the internal volume of the first container. If the volume of the liquid filled in the first container exceeds 85% by volume, the problem of liquid leakage may occur as described above, and since the volume of the initial gas is small, and thus the volume variation rate of gas according to filling of the second container is large, stability in forming a uniform composition of the liquid may be reduced. Since the volume of the liquid in the first container is gradually reduced as the second container is filled with the liquid, the upper limit of the above-described volumes of the liquid may be importantly applied when the nicotine-containing solution and the propellant are initially filled into the first container.

To increase the ratio of liquid propellant actually filled in the second container and can be utilized, among the filled propellants, and to prevent the first container from being unnecessarily large, it may be preferable to allow the volume of the liquid in the first container immediately before the liquid is filled into the second container after the first container is filled with the nicotine-containing solution and the propellant (i.e., a state in which the liquid and gas in the first container are in dynamic equilibrium) to exceed a predetermined level. According to an embodiment of the present invention, the volume of the liquid in the first container immediately before the liquid is filled in the second container after the first container is filled with the nicotine-containing solution and the propellant is 50% by volume or more, specifically 55% by volume or more, and more specifically 60% by volume or more, based on the internal volume of the first container. If the volume of the liquid in the first container is less than 50% by volume, availability of the propellant filled as described above may be remarkably reduced.

The first container is pressurized at a pressure higher than the atmospheric pressure so that the propellant filled in the container may be separated into liquid and gas. Since the liquid propellant is filled into the second container to serve a direct role in carrying aerosol, the first container, immediately after the filling, is pressurized so that a large amount of liquid propellant may exist relative to the gas propellant. The gas propellant finally remains in the first container, and provides propulsion when the liquid in the first container is filled into the second container, which is sufficient if the first container can operate at a predictable level by the Equation for the composition to be described later, and may be adjusted considering the volume ratio range of the liquid in the first container described above. According to an embodiment of the present invention, the gas propellant immediately before the liquid is filled into the second container after the first container is filled with the nicotine-containing solution and the propellant (i.e., a state in which the liquid and gas in the first container are in dynamic equilibrium) is contained in an amount of 5% by weight or less, specifically 3% by weight or less, and more specifically 1% by weight, based on the total weight of the propellant. If the gas propellant exceeds 5% by weight, the content of liquid propellant to be filled in the second container is small, and thus functionality for carrying aerosol according to the input of the propellant is reduced.

The composition of the liquid in the first container can be predicted through an ideal gas equation. Since the nicotine-containing solution filled together with the propellant exists in a liquid state at room temperature, it has significantly lower vapor pressure relative to the propellant that exists in a gas state at room temperature. Further, since the propellant has a low affinity with the nicotine-containing solution, the composition of the liquid in the first container that is predicted based on the vapor pressure of the propellant does not differ greatly from the actual measurement value. Since the composition of the nicotine-containing solution does not change significantly according to the pressure in the first container, the composition of the liquid in the first container can be grasped by calculating the amount of the liquid propellant. First, the volume of the gas propellant can be calculated from the Equation 1 below:

[Equation 1]

$$V_v = \frac{m - (V - V_{l2}) * \rho_l}{\frac{MP_{sat}}{RT} - \rho_l}$$

wherein, $V_v$ is the volume (ml) of gas propellant, m is the amount (g) of filled propellant, V is the capacity (ml) of first container, $V_{l2}$ is the volume (ml) of nicotine-containing solution, $\rho_l$ is the density ($kg/m^3$) of a liquid propellant, M is the molecular weight (g/mol) of propellant, $P_{sat}$ is the saturated vapor pressure (atm), R is a gas constant of 0.082 (atm/(mol·K), and T is temperature (K).

Based on the volume of the gas propellant calculated above, the volume of the liquid propellant may be calculated from Equation 2 below:

[Equation 2]

$$V_l = V - V_{l2} - V_v$$

wherein, V, $V_{l2}$, and $V_v$ are the same as those defined in the Equation 1 above and $V_l$ is the volume (ml) of liquid propellant.

Based on the volume of the liquid propellant calculated above, the weight of the liquid propellant may be calculated from Equation 3 below:

[Equation 3]

$$m_l = \rho_l * V_l$$

wherein, $\rho_l$ and $V_l$ are the same as those defined in Equations 1 and 2 above, and $m_l$ is the mass (g) of liquid propellant.

Based on the mass of the liquid propellant calculated above, the liquid mass of the first container may be calculated from Equation 4 below:

[Equation 4]

$$m_{l3} = m_l + m_{l2}$$

wherein, $m_l$ is the same as that defined in Equation 3 above, $m_{l2}$ is the mass (g) of nicotine-containing solution, and $m_{l3}$ is the mass (g) of liquid in the first container.

As confirmed by the embodiment below, considering that the amount of nicotine in the second container is not changed significantly while filling the second container about 20 times, the aerosol inhaler according to an embodiment of the present invention can be freely designed in the amount of nicotine transition by changing the composition of liquid. The conditions of design described above are helpful within a range predicted when actually implementing the aerosol inhaler.

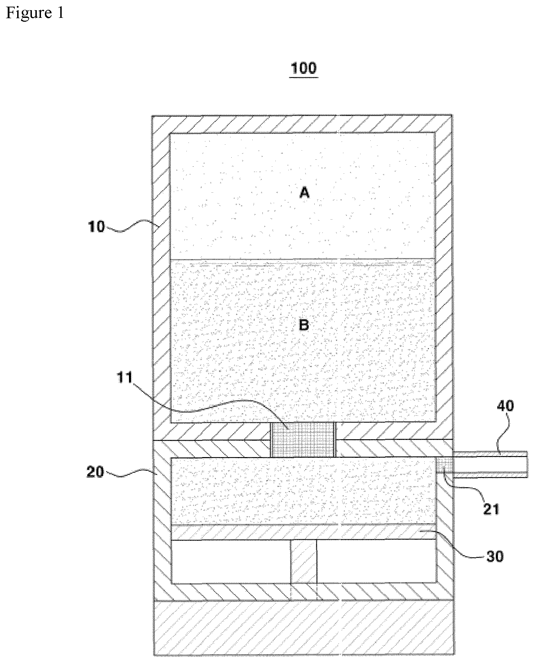

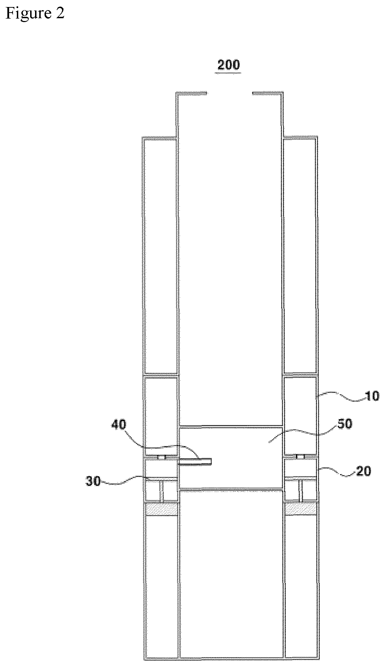

The present specification provides FIGS. 1 and 2 to describe in more detail the aerosol inhaler according to an embodiment of the present invention. FIG. 1 is a view schematically showing the structure of a nicotine transition unit according to an embodiment of the present invention, and FIG. 2 is a view schematically showing the structure of an aerosol inhaler including the nicotine transition unit according to an embodiment of the present invention, which are only one exemplary structure and the present invention is not limited thereto.

As shown in FIG. 1, a nicotine transition unit 100 includes a first container 10 and a second container 20, wherein the first container 10 and the second container 20 are connected by a connection channel 11. The first container 10 is filled with nicotine-containing solution and propellant, and after the liquid and gas are in dynamic equilibrium inside the first container 10, the connection channel 11 is opened and the liquid flows into the second container 20 through the connection channel 11 to fill the second container 20 with the liquid of the first container. After the inside of the second container 20 is completely filled, the connection channel 11 is closed to separate the second container 20 from the first container 10. The opening and closing means of the connection channel 11 may be appropriately applied by means generally known in the relevant technical field, such as valves, caps, and the like.

According to an embodiment of the present invention, when the second container is filled with the liquid of the first container, the internal space of the second container 20 is contracted and relaxed according to the amount of the liquid filled in the first container so that air in the second container does not flow into the first container 10. Contraction and relaxation of the internal space may be implemented by contraction and relaxation of the container itself, or may be implemented by a contraction/relaxation means 30 if the container itself is in a fixed form as shown in FIG. 1. The contraction/relaxation means 30 in FIG. 1 moves downward to relax the internal space of the second container 20, thereby filling the liquid of the first container into the second container 20, and moves upward to contract the internal space of the second container 20, thereby discharging the liquid filled in the second container 20. The discharge of the liquid filled in the second container 20 may be performed through a delivery pipe 40 connected to an aerosol generator 50 to be described later. When the liquid is filled in the second container 20, the connection channel 11 between the first container and the second container is opened, and the connection channel 21 between the second container and the delivery pipe is closed. On the contrary, when the liquid in the second container 20 is discharged, the connection channel 11 between the first container and the second container is closed, and the connection channel 21 between the second container and the delivery pipe is opened. As shown in FIG. 1, as the contraction/relaxation means 30 moves downward, only liquid B, not gas A, may be filled into the second container 20.

As shown in FIG. 2, an aerosol inhaler 200 further includes an aerosol generator 50. The aerosol generator 50 is connected to the second container 20 separated from the first container 10, is supplied with the liquid filled in the second container, and generates aerosol until the liquid is all exhausted. As described above, the aerosol generator 50 may be connected to the second container 20 through the delivery pipe 40 and may be connected to or separated from the second container by opening or closing the connection channel 21 between the second container and the delivery pipe 21.

The second container 20 may include the liquid to a degree suitable for using one time by the user. According to an embodiment of the present invention, the liquid filled in the second container 20 is such an amount that the aerosol generated through the aerosol generator may be all exhausted by 5 to 15 puffs, specifically 7 to 14 puffs, and more specifically 8 to 12 puffs. The above range is close to a standard used by the user at one time in the relevant technical field.

When the liquid filled in the second container 20 is all exhausted, the second container 20 is separated from the aerosol generator 50 and is connected to the first container 10 to be filled with the liquid of the first container. The filling and exhaustion of liquid in the second container 20 may be repeated several times to several tens of times. However, if continuously filling the liquid into the second container 20 until the liquid in the first container 10 is all exhausted, the liquid filled in the second container 20 around the time when the liquid in the first container 10 is all exhausted may have a significant difference from a predicted value calculated through the ideal gas equation described above in the composition of liquid. Accordingly, if the liquid in the first container 10 is exhausted to a predetermined reference or less, it may be preferable to stop filling the second container 20. According to an embodiment of the present invention, the repeating process of filling and exhausting the liquid is stopped when the volume of the liquid in the first container is 15% by volume or less, specifically 13% by volume or less, and more specifically 11% by volume or less, based on the internal volume of the first container. By stopping the filling into the second container 20 within the above range, it is possible to control so that a change in the amount of nicotine transition is not significant at every time of filling. According to an embodiment of the present invention, the liquid in the first container is such an amount that the second container can be filled 15 time to 25 times until the filling is stopped. It may be preferable in terms of availability of the aerosol inhaler to control the amount of liquid in the first container to within the above range.

## Example

Into a 19 ml cylindrical canister (about 21 mm in diameter and about 53 mm in height), 18.679 g of 1,1,1,2-tetrafluoroethane (product by Xiamen Juda Chemical & Equipment company) and 0.321 g of nicotine-containing liquid were filled, and then the canister was sealed. The nicotine-containing liquid was prepared by mixing 13.5 mg of nicotine (product by JHchemical company) into a mixed solution (3:1 weight ratio) of ethanol (product by Sigma-Aldrich company) and glycerol (product by SK company). After the liquid and gas inside the sealed canister were in dynamic equilibrium, 0.8 g of liquid was extracted and filled in an imitation tobacco storage.

After the liquid filled in the imitation tobacco storage was inhaled and completely exhausted, 0.8 g of liquid (an amount that can be divided and inhaled by 10 to 12 puffs) was extracted from the sealed canister where the liquid and gas were in dynamic equilibrium to fill it again into the imitation tobacco storage for the second time. After filling the imitation tobacco a total of 20 times according to the process described above, the cylindrical canister (Table 1) and the components of extracted liquid (Table 2) at each filling were analyzed and shown in Tables 1 and 2 below. For reference, the initial volume of liquid in the canister is 81% by volume, and the volume of liquid in the canister after filling 20 times is 11% by volume.

[Table 1]

| Number of filling | Amount of liquid in canister (g) | Amount of liquid propellant (g) | Amount of gas propellant (g) | Amount of nicotine-containing liquid (g) | Total amount of filling in canister (g) |
|---|---|---|---|---|---|
| Early stage | 18.90 | 18.58 | 0.10 | 0.321 | 19.000 |
| 1 | 18.08 | 17.77 | 0.12 | 0.307 | 18.200 |
| 2 | 17.26 | 16.97 | 0.14 | 0.294 | 17.400 |
| 3 | 16.44 | 16.16 | 0.16 | 0.280 | 16.600 |
| 4 | 15.63 | 15.36 | 0.17 | 0.267 | 15.800 |
| 5 | 14.81 | 14.55 | 0.19 | 0.253 | 15.000 |
| 6 | 13.99 | 13.75 | 0.21 | 0.239 | 14.200 |
| 7 | 13.17 | 12.94 | 0.23 | 0.226 | 13.400 |
| 8 | 12.35 | 12.14 | 0.25 | 0.212 | 12.600 |
| 9 | 11.53 | 11.33 | 0.27 | 0.198 | 11.800 |
| 10 | 10.71 | 10.53 | 0.29 | 0.184 | 11.000 |
| 11 | 9.89 | 9.72 | 0.31 | 0.171 | 10.200 |
| 12 | 9.08 | 8.92 | 0.32 | 0.157 | 9.400 |
| 13 | 8.26 | 8.11 | 0.34 | 0.143 | 8.600 |
| 14 | 7.44 | 7.31 | 0.36 | 0.129 | 7.800 |
| 15 | 6.62 | 6.51 | 0.38 | 0.115 | 7.000 |
| 16 | 5.80 | 5.70 | 0.40 | 0.101 | 6.200 |
| 17 | 4.98 | 4.90 | 0.42 | 0.087 | 5.400 |
| 18 | 4.16 | 4.09 | 0.44 | 0.073 | 4.600 |
| 19 | 3.35 | 3.29 | 0.45 | 0.059 | 3.800 |
| 20 | 2.53 | 2.48 | 0.47 | 0.045 | 3.000 |

[Table 2]

| Number of filling | Amount of extraction of liquid (g) | Amount of propellant (g) | Amount of nicotine-containing liquid (g) | Amount of nicotine (g) |
|---|---|---|---|---|
| 1 | 0.8 | 0.7864 | 0.0136 | 0.571 |
| 2 | 0.8 | 0.7864 | 0.0136 | 0.572 |
| 3 | 0.8 | 0.7864 | 0.0136 | 0.573 |
| 4 | 0.8 | 0.7864 | 0.0136 | 0.573 |
| 5 | 0.8 | 0.7864 | 0.0136 | 0.574 |
| 6 | 0.8 | 0.7863 | 0.0137 | 0.575 |
| 7 | 0.8 | 0.7863 | 0.0137 | 0.575 |
| 8 | 0.8 | 0.7863 | 0.0137 | 0.576 |
| 9 | 0.8 | 0.7863 | 0.0137 | 0.577 |
| 10 | 0.8 | 0.7863 | 0.0137 | 0.578 |
| 11 | 0.8 | 0.7862 | 0.0138 | 0.579 |
| 12 | 0.8 | 0.7862 | 0.0138 | 0.580 |
| 13 | 0.8 | 0.7862 | 0.0138 | 0.581 |
| 14 | 0.8 | 0.7861 | 0.0139 | 0.583 |
| 15 | 0.8 | 0.7861 | 0.0139 | 0.584 |
| 16 | 0.8 | 0.7861 | 0.0139 | 0.586 |
| 17 | 0.8 | 0.7860 | 0.0140 | 0.588 |
| 18 | 0.8 | 0.7860 | 0.0140 | 0.590 |
| 19 | 0.8 | 0.7859 | 0.0141 | 0.592 |
| 20 | 0.8 | 0.7858 | 0.0142 | 0.596 |

According to Tables 1 and 2 above, it was confirmed that as the number of filling the imitation tobacco storage was increased, the amount of the liquid propellant having high content was decreased more rapidly than in the liquid, and accordingly even in the extracted liquid composition, the amount of propellant was decreased and the amount of nicotine-containing liquid was increased. Nonetheless, if comparing the content of nicotine between the initially extracted liquid composition and the liquid composition extracted at the 20th time, since only a 0.025 mg (about 4.4%) was increased from the initial 0.571 mg to 0.596 mg at 20th time, when designing the nicotine transition unit in this way, it is possible to quantitatively inhale within 5% of the change in the nicotine transition amount.

As described above, although the embodiments were described with reference to the limited embodiments and drawings, they may be changed and modified in various ways by those skilled in the art. For example, the described technologies may be performed in an order different from the described method, and/or even if the components such as the described system, structure, device, and circuit, etc. are connected or combined in a form different from the described method, or replaced or substituted by other components or equivalents, appropriate results may be achieved.

**Description of Reference Numerals**

10: first container
11: connection channel between first container and second container
20: second container
21: connection channel between second container and delivery pipe
30: contraction/relaxation means (for internal space of second container)
40: delivery pipe
50: aerosol generator
100: nicotine transition unit

200: aerosol inhaler
A: gas
B: liquid

**Claims**

1. An aerosol inhaler comprising a nicotine transition unit (100), wherein the nicotine transition unit (100) includes:

    a first container (10) filled with a nicotine-containing solution and a propellant; and
    a second container (20) connected to the first container (10) to be filled with the liquid of the first container (10), and
    the propellant exists in a liquid state and a gas state in the first container (10),
    **characterized in that**
    an internal space of the second container (20) is contracted and relaxed according to the amount filled with the liquid of the first container (10).

2. The aerosol inhaler of claim 1, wherein the propellant is a substance having vapor pressure of approximately 413.7 kPa relative to ambient atmospheric pressure to 689.5 kPa relative to ambient atmospheric pressure at 21°C.

3. The aerosol inhaler of claim 1, wherein the nicotine-containing solution in the first container (10) is filled in an amount of 1% to 10% by weight based on the total weight of the propellant.

4. The aerosol inhaler of claim 1, wherein the volume of the liquid comprising the nicotine-containing solution and the liquid propellant in the first container (10) is 85% by volume or less based on the internal volume of the first container (10).

5. The aerosol inhaler of claim 1, wherein the gas propellant immediately before the second container (20) is filled with the liquid after the first container (10) is filled with the nicotine-containing solution and the propellant is contained in an amount of 5% by weight or less based on the total weight of the propellant.

6. The aerosol inhaler of claim 1, wherein the second container (20) is filled with the liquid of the first container (10), and is separated from the first container (10) after the inside is completely filled.

7. The aerosol inhaler of claim 6, further comprising an aerosol generator (50), wherein the aerosol generator (50) is connected to the second container (20) separated from the first container (10), is supplied with the liquid filled in the second container (20), and generates aerosol until the liquid is all exhausted.

8. The aerosol inhaler of claim 7, wherein the liquid filled in the second container (20) is such an amount that the aerosol generated through the aerosol generator (50) may be all exhausted by 5 puffs to 15 puffs.

9. The aerosol inhaler of claim 7, wherein when the liquid filled in the second container (20) is all exhausted, the second container (20) is separated from the aerosol generator (50), is connected to the first container (10), and is filled with the liquid of the first container (10), and a repeating process of filling and exhausting the liquid is stopped when the volume of the liquid in the first container (10) is 15% by volume or less based on the internal volume of the first container (10).

10. The aerosol inhaler of claim 9, wherein the liquid in the first container (10) is such an amount that the second container (20) can be filled 15 times to 25 times until the filling is stopped.

**Patentansprüche**

1. Aerosolinhalationseinrichtung, die eine Nikotinübergangseinheit (100) enthält, wobei die Nikotinübergangseinheit (100) Folgendes umfasst:

    einen ersten Behälter (10), der mit einer nikotinhaltigen Lösung und einem Treibmittel gefüllt ist; und
    einen zweiten Behälter (20), der mit dem ersten Behälter (10) verbunden wird, damit er mit der Flüssigkeit des ersten Behälters (10) befüllt wird,

wobei das Treibmittel im ersten Behälter (10) in einem flüssigen Zustand und einem Gaszustand vorhanden ist, **dadurch gekennzeichnet, dass**
ein Innenraum des zweiten Behälters (20) gemäß der Menge, die mit der Flüssigkeit des ersten Behälters (10) befüllt wird, zusammengezogen und entspannt wird.

2. Aerosolinhalationseinrichtung nach Anspruch 1, wobei das Treibmittel eine Substanz ist, die einen Dampfdruck von näherungsweise 413,7 kPa bei einem Umgebungsatmosphärendruck von 689,5 kPa bei einem Umgebungsatmosphärendruck von 21 °C aufweist.

3. Aerosolinhalationseinrichtung nach Anspruch 1, wobei die nikotinhaltige Lösung im ersten Behälter (10) in einer Menge im Bereich von 1 Gew.-% bis 10 Gew.-% basierend auf dem Gesamtgewicht des Treibmittels eingefüllt ist.

4. Aerosolinhalationseinrichtung nach Anspruch 1, wobei das Volumen der Flüssigkeit, die die nikotinhaltige Lösung und das flüssige Treibmittel umfasst, im ersten Behälter (10) 85 Vol.-% oder weniger basierend auf dem Innenvolumen des ersten Behälters (10) umfasst.

5. Aerosolinhalationseinrichtung nach Anspruch 1, wobei das gasförmige Treibmittel unmittelbar bevor der zweite Behälter (20) mit der Flüssigkeit befüllt wird, nachdem der erste Behälter (10) mit der nikotinhaltigen Lösung und dem Treibmittel befüllt worden ist, in einer Menge von 5 Gew.-% oder weniger basierend auf dem Gesamtgewicht des Treibmittels enthalten ist.

6. Aerosolinhalationseinrichtung nach Anspruch 1, wobei der zweite Behälter (20) mit der Flüssigkeit des ersten Behälters (10) befüllt wird und vom ersten Behälter (10) getrennt wird, nachdem der Innenraum vollständig befüllt worden ist.

7. Aerosolinhalationseinrichtung nach Anspruch 6, die ferner einen Aerosolgenerator (50) umfasst,
wobei der Aerosolgenerator (50) mit dem zweiten Behälter (20) verbunden wird, der vom ersten Behälter (10) getrennt ist, mit der Flüssigkeit versorgt wird, die im zweiten Behälter (20) eingefüllt ist, und Aerosol erzeugt, bis die Flüssigkeit vollständig verbraucht ist.

8. Aerosolinhalationseinrichtung nach Anspruch 7, wobei die Flüssigkeit, die in den zweiten Behälter (20) eingefüllt wird, in einer Menge vorliegt, derart, dass das Aerosol, das durch den Aerosolgenerator (50) erzeugt wird, durch 5 Züge bis 15 Züge vollständig verbraucht werden kann.

9. Aerosolinhalationseinrichtung nach Anspruch 7, wobei dann, wenn die Flüssigkeit, die im zweiten Behälter (20) eingefüllt ist, vollständig verbraucht ist, der zweite Behälter (20) vom Aerosolgenerator (50) getrennt wird, mit dem ersten Behälter (10) verbunden wird und mit der Flüssigkeit des ersten Behälters (10) befüllt wird, und
ein Wiederholungsprozess des Befüllens und Verbrauchens der Flüssigkeit angehalten wird, wenn das Volumen der Flüssigkeit im ersten Behälter (10) 15 Vol.-% oder weniger basierend auf dem Innenvolumen des ersten Behälters (10) ist.

10. Aerosolinhalationseinrichtung nach Anspruch 9, wobei die Flüssigkeit im ersten Behälter (10) in einer Menge vorliegt, derart, dass der zweite Behälter (20) 15-mal bis 25-mal befüllt werden kann, bis das Befüllen angehalten wird.

**Revendications**

1. Inhalateur d'aérosol comportant une unité de transition de nicotine (100), dans lequel l'unité de transition de nicotine (100) inclut :

un premier contenant (10) rempli d'une solution contenant de la nicotine et d'un agent propulseur ; et
un second contenant (20) relié au premier contenant (10) à remplir du liquide du premier contenant (10), et
l'agent propulseur se trouve dans un état liquide et un état gazeux dans le premier contenant (10),
**caractérisé en ce que**
un espace interne du second contenant (20) est comprimé et détendu en fonction de la quantité de remplissage avec le liquide du premier contenant (10).

2. Inhalateur d'aérosol selon la revendication 1, dans lequel l'agent propulseur est une substance ayant une pression de vapeur d'approximativement 413,7 kPa par rapport à une pression atmosphérique ambiante à 689,5 kPa par rapport à une pression atmosphérique ambiante à 21 °C.

3. Inhalateur d'aérosol selon la revendication 1, dans lequel la solution contenant de la nicotine remplit le premier contenant (10) à une quantité de 1 % à 10 % en poids sur la base du poids total de l'agent propulseur.

4. Inhalateur d'aérosol selon la revendication 1, dans lequel le volume du liquide comportant la solution contenant de la nicotine et l'agent propulseur liquide dans le premier contenant (10) est de 85 % en volume ou moins sur la base du volume interne du premier contenant (10).

5. Inhalateur d'aérosol selon la revendication 1, dans lequel l'agent propulseur gazeux juste avant le remplissage du second contenant (20) avec le liquide après le remplissage du premier contenant (10) avec la solution contenant de la nicotine et l'agent propulseur est contenu en une quantité de 5 % en poids ou moins sur la base du poids total de l'agent propulseur.

6. Inhalateur d'aérosol selon la revendication 1, dans lequel le second contenant (20) est rempli du liquide du premier contenant (10) et est séparé du premier contenant (10) après le remplissage complet de l'intérieur.

7. Inhalateur d'aérosol selon la revendication 6, comportant en outre un dispositif de production d'aérosol (50), dans lequel le dispositif de production d'aérosol (50) est relié au second contenant (20) séparé du premier contenant (10), est alimenté en liquide remplissant le second contenant (20) et produit un aérosol jusqu'à ce que la totalité du liquide soit épuisée.

8. Inhalateur d'aérosol selon la revendication 7, dans lequel le liquide remplissant le second contenant (20) est présent en une quantité telle que l'aérosol produit à travers le dispositif de production d'aérosol (50) peut être entièrement épuisé en 5 à 15 bouffées.

9. Inhalateur d'aérosol selon la revendication 7, dans lequel, lorsque le liquide remplissant le second contenant (20) est entièrement épuisé, le second contenant (20) est séparé du dispositif de production d'aérosol (50), est relié au premier contenant (10) et est rempli du liquide du premier contenant (10), et
un processus répété de remplissage et d'épuisement du liquide est arrêté lorsque le volume du liquide dans le premier contenant (10) est de 15 % en volume ou moins sur la base du volume interne du premier contenant (10).

10. Inhalateur d'aérosol selon la revendication 9, dans lequel le liquide dans le premier contenant (10) est en une quantité telle que le second contenant (20) peut être rempli 15 fois à 25 fois jusqu'à ce que le remplissage soit arrêté.

Figure 1

Figure 2

**EP 4 169 397 B1**

**Patent documents cited in the description**

- KR 101257597
- WO 2012045683 A2
- KR 20010080091 A